# EUROPEAN PATENT APPLICATION

(11) **EP 4 799 599 A2**
(43) Date of publication of application: **02.09.2026**
(21) Application number: 26178752.7
(22) Date of filing: 01.10.2015
(51) Int. Cl.: A61J 3/10

(54) **PHARMACEUTICAL COMPOSITIONS COMPRISING ALPELISIB**

(30) Priority: 03.10.2014 US 201462059331 P
(62) Document number(s) of the earlier application(s) in accordance with Art. 76 EPC: 22187029.8 / 4 169 508; 15778761.5 / 3 200 772
(71) Applicant: Novartis AG, 4056 Basel (CH)
(72) Inventor: Bock, Michaela Anna Maria, 4002 Basel (CH); Elbaz, Frantz, 4056 Basel (CH); Kochhar, Charu, 4002 Basel (CH); Stingelin, Doris, 4002 Basel (CH)
(74) Representative: Carpmaels & Ransford LLP

(57) **Abstract**

The present invention relates to dispersible tablets comprising the compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof.

## Description

### Field of the Invention

The present invention relates to novel dispersible tablets comprising the compound *(S)-*Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof.

### Background of the Invention

Phosphatidylinositol 3-kinases ("PI-3 kinase" or "PI3K") comprise a widely expressed family of lipid kinases that function as key signal transducers downstream of cell-surface receptors and key regulators of cell metabolism and survival.

Of the two Class I PI3Ks, Class IA PI3Ks are heterodimers composed of a catalytic p110 subunit (α, β, δ isoforms) constitutively associated with a regulatory subunit that can be p85α, p55α, p50α, p85β or p55γ. These catalytic p110 subunits (α, β, δ isoforms) are encoded by three genes (PIK3CA, PIK3CB and PIK3CD) in humans. Class 1B PI3K has one family member, a heterodimer composed of a catalytic p110γ subunit associated with one of two regulatory subunits, either the p101 or the p84. The modular domains of the p85/55/50 subunits include Src Homology (SH2) domains that bind phosphotyrosine residues in a specific sequence context on activated receptor and cytoplasmic tyrosine kinases, resulting in activation and localization of Class IA PI3Ks. Class IB, as well as p110β in some circumstances, is activated directly by G protein-coupled receptors that bind a diverse repertoire of peptide and non-peptide ligands (Stephens et al., Cell 89:105 (1997)); Katso et al., Annu. Rev. Cell Dev. Biol. 17:615-675 (2001)). Consequently, resultant phospholipid products of class I PI3K link upstream receptors with downstream cellular activities including proliferation, survival, chemotaxis, cellular trafficking, motility, metabolism, inflammatory and allergic responses, transcription and translation (Cantley et al., Cell 64:281 (1991); Escobedo and Williams, Nature 335:85 (1988); Fantl et al., Cell 69:413 (1992)).

Deregulation of PI3K is one of the most common deregulations associated with human cancers and proliferative diseases (Parsons et al., Nature 436:792 (2005); Hennessey at el., Nature Rev. Drug Disc. 4:988-1004 (2005)). The tumor suppressor gene PTEN, which dephosphorylates phosphoinositides at the 3' position of the inositol ring and in so doing antagonizes PI3K activity, is functionally deleted in a variety of tumors. The genes for the p110α isoform (PIK3CA) is amplified and increased protein expression of its gene product has been demonstrated in several human cancers. Mutations and translocation of p85α that serve to up-regulate the p85-p110 complex have been described in human cancers. Finally, somatic missense mutations in PIK3CA that activate downstream signaling pathways have been described at significant frequencies in a wide diversity of human cancers, including 32% of colorectal cancers, 27% of glioblastomas, 25% of gastric cancers, 36% of hepatocellular carcinomas, and 18-40% of breast cancers.

(S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) is a specific 2-carboxamide cycloamino urea derivative compound that potently and selectively targets the alpha (a)-isoform of class IA PI3K. This compound has the following chemical structure: 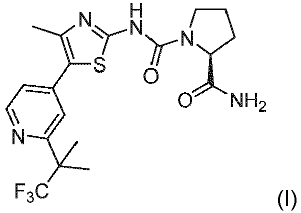 (hereinafter, "Compound I"). Compound I has demonstrated clinical efficacy in the single agent treatment of patients having advanced solid malignancies carrying an alteration in the PIK3CA gene in a Phase I clinical trial. As of March 10, 2014, out of the 131 patients evaluable for radiologic response, 15 patients with PIK3CA-altered solid tumors had partial response to treatment with Compound I and 68 patients had stable disease, as defined by Response Evaluation Criteria in Solid Tumors (RECIST) guideline version 1.0 criteria (Therasse P. et al., "New Guidelines to Evaluate the Response to Treatment in Solid Tumors", JNCI National Cancer Inst. (2000), 2(3): 205-216.). Further, Compound I has demonstrated in vivo dose-dependent antitumor activity in PIK3CA-mutant or amplified tumor xenograft models, such as breast, head and neck, and ovarian cancers. Currently, Compound I is being evaluated in a Phase Ib/II clinical trial in combination with cetuximab for the treatment of patients with recurrent or metastatic head and neck squamous cell carcinoma (NCT01602315).

Depending on age, patient condition, mode of administration, and type of disease, Compound I or a pharmaceutically acceptable salt thereof is orally administered at an effective daily dosage of about 1 to 6.5 mg/kg in adults or children. In a 70 kg body weight adult patient, Compound I or a pharmaceutically acceptable salt thereof is orally administered at a daily dosage of about 70 mg to 455 mg. Compound I or a pharmaceutically acceptable salt thereof is currently orally administered to patients once or twice daily in one or more solid tablets swallowed by said patient. These dosage forms provide efficacious administration of this agent, are convenient to administer, and are stable. However, for certain groups of patients, oral administration of medicaments in solid tablet form is either undesirable or impractical. In particular, children, elderly patients and patients suffering from head and neck cancers may be unable to swallow such tablets conveniently. For these patients, it is typically desirable or necessary to provide alternative dosage forms or alternative methods for administering a medicament.

It has been surprisingly found that Compound I or a pharmaceutically acceptable salt thereof may be formulated in form of the novel dispersible tablet of the present invention with high drug loading (e.g., from about 5% to 50%, preferably about 35% to 45%, weight based on the total weight of the tablet) and which disperses in aqueous medium in 5 minutes or less, preferably 3 minutes or less and which produces a smooth dispersion which can pass through a sieve screen with a nominal mesh aperture of 710µm. This novel dispersible tablet also allows Compound I or a pharmaceutically acceptable salt thereof to be conveniently and safely administered to patients with swallowing difficulties at a pharmacologically active daily dosage amount of Compound I.

### Summary of the Invention

The present invention provides a dispersible tablet comprising (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) at least one disintegrant in a total amount of about 1% to 25% in weight based on the total weight of the tablet, (c) at least one binder in a total amount of about 1% to 20% in weight based on the total weight of the tablet, (d) at least one lubricant in a total amount of about 1% to 15% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

Preferably, the dispersible tablet of the present invention comprises granulates having an inner phase and outer phase, wherein the inner phase comprises *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant, at least one binder, and optionally any additional excipients, wherein the outer phase comprises at least one disintegrant, at least one lubricant and optionally any additional excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

In one embodiment, the present invention relates to a dispersible tablet comprising (a) *(S)-*Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) sodium starch glycolate in an amount of about 2% to 12% in weight based on the total weight of the tablet, (c) low-substituted hydroxypropyl cellulose in an amount of about 1% to 10 % in weight based on the total weight of the tablet, and (d) sodium stearyl fumarate in an amount of about 1% to 7 % in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

In one embodiment, the present invention relates to a dispersible tablet comprising (a) (*S)-*Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 35% to 45% in weight based on the total weight of the tablet, (b) sodium starch glycolate in an amount of about 5% to 10% in weight based on the total weight of the tablet, (c) low-substituted hydroxypropyl cellulose in an amount of about 1% to 3% in weight based on the total weight of the tablet, and (d) sodium stearyl fumarate in an amount of about 3% to 5% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

In one embodiment, the dispersible tablets of the present invention is administered to a patient, such as a child or an adult suffering from a proliferative disease (e.g., a cancer), by (i) contacting said tablet with an ingestible liquid, (ii) allowing the tablet to disperse in the ingestible liquid to form a dispersed mixture, e.g., in 5 minutes or less, preferably 3 minutes or less, and (iii) ingesting the dispersed mixture.

In one embodiment, the dispersible tablets of the present invention may be administered to a patient in need thereof by (i) contacting said tablet with an ingestible liquid (ii) allowing the tablet to disperse in the ingestible liquid to form a dispersed mixture, e.g., in 5 minutes or less, preferably 3 minutes or less, and (iii) administering said dispersed mixture said patient using or via a feeding tube, preferably a gastronomy tube (G-tube) or PEG (percutaneous endoscopic gastrostomy) tube.

In one aspect, the present invention relates to a process for producing a dispersible tablet of the present invention, comprising
(a) forming a granulate having an inner phase and an outer phase by
   (i.) wet granulating an inner phase comprising (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, at least one disintegrant in a total amount of about 1% to 15% in weight based on the total weight of the tablet, at least one binder in a total amount of about 1% to 20% in weight based on the total weight of the tablet, and optionally any additional pharmaceutically acceptable excipients;
   (ii.) adding at least one disintegrant in a total amount of about 1% to 10% in weight based on the total weight of the tablet, and optionally any additional pharmaceutically acceptable excipients to the inner phase formed in step (a)(i.) and mixing; and
   (iii.) adding at least one lubricant in a total amount of about 1% to 15% in weight based on the total weight of the tablet to the mixture formed in step (a)(ii.) and mixing; and
(b) forming the dispersible tablet by compressing the mixture obtained in step (a)(iii.),
with the proviso that any additional pharmaceutically acceptable excipient according to step (a)(i.) or (a)(ii.) is not calcium phosphate tribasic, calcium phosphate dibasic anhydrous, calcium phosphate dibasic dihydrate or sucralose. It is understood that steps (a)(ii) and (a)(iii) form the outer phase of the granulate. In one embodiment, the process further comprises the step of applying a film-coat to the tablet.

In one embodiment, the present invention relates to the dispersible tablets of the present invention for use in the treatment or prevention of a proliferative disorder, preferably a cancer.

In one embodiment, the present invention relates to use of the dispersible tablet of the present invention for the treatment or prevention of a proliferative disease, preferably a cancer.

In one embodiment, the present invention relates to use of the dispersible tablet of the present invention for manufacture of a medicament for the treatment or prevention of a proliferative disease, preferably a cancer.

In one embodiment, the present invention relates to a method of treatment or prevention of a proliferative disease comprising administering to a patient in need thereof one or more dispersible tablets of the present invention that together comprise a therapeutically effective amount of Compound I or a pharmaceutically acceptable salt thereof.

In one embodiment, the present invention further relates to a medicament package comprising dispersible tablets according to the present invention and printed instructions directing one or more dispersible tablets of Compound I or a pharmaceutically acceptable salt thereof be administered orally.

### Detailed Description of the Invention

The present invention provides a dispersible tablet comprising (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) at least one disintegrant in a total amount of about 1% to 25% in weight based on the total weight of the tablet, (c) at least one binder in a total amount of about 1% to 20% in weight based on the total weight of the tablet, (d) at least one lubricant in a total amount of about 1% to 15% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

By "dispersible tablet" is meant an uncoated or film-coated tablet which disperses in aqueous medium, e.g., in water, soda or juice (e.g or vegetable juice), before administration.

Preferably, the dispersible tablet of the present invention comprises granulates having an inner phase and outer phase, wherein the inner phase comprises *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant, at least one binder, and optionally any additional excipients, wherein the outer phase comprises at least one disintegrant, at least one lubricant and optionally any additional excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

The present invention provides a dispersible tablet with high drug loading comprising Compound I or a pharmaceutically acceptable salt thereof as active ingredient. Compound I or a pharmaceutically acceptable salt thereof is present in an amount from about 5% to 50%, e.g., from about 10% to 50% or 20% to 50% or 30% to 45%, preferably 35% to 45% in weight based on the total weight of the dispersible tablet. In particular, the amount of Compound I or a pharmaceutically acceptable salt thereof may range from 35 to 45%, e.g., about 36% to 41%, in weight based on the total weight of the dispersible tablet.

Certain terms used herein are described below. Unless defined otherwise, all technical and scientific terms used herein have the same meaning as is commonly understood by one of skill in the art to which this invention belongs. The following general definitions shall apply in this specification, unless otherwise specified:
The terms "comprising" and "including" are used herein in their open-ended and non-limiting sense unless otherwise noted.

The terms "a" and "an" and "the" and similar references in the context of describing the invention (especially in the context of the following claims) are to be construed to cover both the singular and the plural, unless otherwise indicated herein or clearly contradicted by context. Where the plural form is used for compounds, salts, and the like, this is taken to mean also a single compound, salt, or the like.

The term "pharmaceutically acceptable" is defined herein to refer to those compounds, materials, excipients, compositions and/or dosage forms, which are, within the scope of sound medical judgment, suitable for contact with the tissues a subject, e.g., a mammal or human, without excessive toxicity, irritation allergic response and other problem complications commensurate with a reasonable benefit / risk ratio.

The term "daily dosage" refers to the total dosage amount of the therapeutic agent administered to a specific patient in any single day or twenty-four hour period.

The phrase "effective amount" or "therapeutically effective amount" is used herein to mean an amount sufficient to reduce by at least about 15 percent, preferably by at least 50 percent, more preferably by at least 90 percent, and most preferably prevent, a clinically significant deficit in the activity, function and response of the subject in need thereof. Alternatively, an effective amount or therapeutically effective amount is an amount sufficient to provide an observable improvement over the baseline clinically observable signs and symptoms of a disease.

The term "pharmaceutical composition" is defined herein to refer to a mixture or solution containing at least one active ingredient to be administered to a subject, e.g., a mammal or human, in order to prevent or treat a particular disease or condition affecting the subject.

By "inner phase" is meant the granulate phase or core including the active ingredient *(S)-*Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant, at least one binder, and optionally one or more additional pharmaceutically acceptable excipients. It is also known as the internal phase.

By "outer phase" is meant at least one disintegrant, at least one lubricant, and optionally one or more additional pharmaceutically acceptable excipients which are added to the inner phase (granulates). It is also known as the external phase.

By "total weight of the dispersible tablet" is meant the weight of a tablet being the inner and outer phase.

The term "about" or "approximately" shall have the meaning of within 10%, more preferably within 5%, of a given value or range.

The term "subject" refers to an animal. Typically the animal is a mammal. A subject also refers to for example, primates (e.g., humans), cows, sheep, goats, horses, dogs, cats, rabbits, rats, mice, fish, birds and the like. In certain embodiments, the subject is a primate. In yet other embodiments, the subject is a human.

The present invention relates to a dispersible tablet comprising (a) (*S)-*Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) at least one disintegrant in a total amount of about 1% to 25% in weight based on the total weight of the tablet, (c) at least one binder in a total amount of about 1% to 20% in weight based on the total weight of the tablet, (d) at least one lubricant in a total amount of about 1% to 15% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

Preferably, the dispersible tablet of the present invention comprises granulates having an inner phase and outer phase, wherein the inner phase comprises *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant, at least one binder, and optionally any additional excipients, wherein the outer phase comprises at least one disintegrant and optionally any additional excipients.

*(S)*-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) ("Compound I") is a specific 2-carboxamide cycloamino urea derivative compound that potently and selectively targets the alpha (α)-isoform of class IA PI3K and having the following chemical structure: 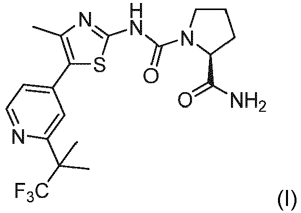 Compound I and its pharmaceutically acceptable salts are described in PCT Application No. WO2010/029082, which is hereby incorporated by reference in its entirety, and methods of its preparation have been described, for example, in Example 15 therein. Preferably, Compound I is in the free base form.

Compound I may be orally administered at an effective daily dose of about 1 to 6.5 mg/kg in human adults or children. Compound I may be orally administered to a 70 kg body weight human adult at a daily dosage of about 70 mg to 455 mg, e.g, about 200 to 400 mg, or about 240 mg to 400 mg, or about 300 mg to 400 mg, or about 350 mg to 400 mg, in a single dose or in divided doses up to four times a day. Preferably, Compound I is administered to a 70 kg body weight human adult at a daily dosage of about 350 mg to about 400 mg.

The present invention relates to a dispersible tablet with high drug loading comprising Compound I as active ingredient. Compound I is present in an amount from about 5% to 50%, e.g., from about 10% to 50% or about 20% to 50% or about 30% to 45%, preferably about 35% to 45% in weight based on the total weight of the dispersible tablet. In particular, the amount of Compound I may range from about 35 to 45%, e.g., about 36% to 41%, in weight based on the total weight of the dispersible tablet.

Suitable disintegrants for the dispersible tablets of the present invention include but are not limited to microcrystalline cellulose, sodium starch glycolate, low-substituted hydroxypropyl cellulose, sodium croscarmellose, calcium croscarmellose, cross-linked polyvinylpyrrolidone (e.g., as commercially available under the tradenames Crospovidone^{®} or Polyplasdone^{®} or Kollidone^{®}CL), cross-linked alginic acid, sodium alginate, potassium alginate, gellan gum, corn starch, pregelatinized starch, carboxymethylcellulose sodium, glycine and any combination thereof. Preferably, sodium starch glycolate or low -substituted hydroxypropyl cellulose is used. More preferably, both sodium starch glycolate and low-substituted hydroxypropyl cellulose are used.

In the dispersible tablets of the present invention, the disintegrant is present in a total amount from about 1 % to 25%, preferably about 2% to 12%, e.g., about 5% to 12%, e.g., about 5 to 10% in weight based on the total weight of the tablet.

Thus, the disintegrant may be sodium starch glycolate which is present in a total amount of about 2% to 12%, preferably about 5% to 10%, in weight based on the total weight of the tablet. The disintegrant may also be a mixture of sodium starch glycolate which is present in a total amount of about 5% to 10% in weight based on the total weight of the tablet and low-substituted hydroxypropyl cellulose which is present in a total amount of about 2% to 5% in weight based on the total weight of the tablet.

In one embodiment, the disintegrant is sodium starch glycolate which is present in a total amount of about 2% to 12%, preferably about 5% to 10%, in weight based on the total weight of the tablet.

In one embodiment, the disintegrant consists of sodium starch glycolate which is present in a total amount of about 5% to 10% in weight based on the total weight of the tablet and low-substituted hydroxypropyl cellulose which is present in a total amount of about 2% to 5% in weight based on the total weight of the tablet.

Suitable binders for the dispersible tablets of the present invention include but are not limited to microcrystalline cellulose, low-substituted hydroxypropyl cellulose, hydroxypropylmethyl cellulose, hydroxymethylcellulose, hydroxyethyl cellulose, starch 1500, gum guar, gum xanthan, polyvinylpyrrolidone, sodium alginate and any combination thereof. Preferably, low-substituted hydroxypropyl cellulose is used.

In the dispersible tablets of the present invention, the binder is present in a total amount from about 1% to 20%, preferably about 1% to 10%, e.g., about 2% to 5%, e.g., about 1% to 3%, in weight based on the total weight of the tablet.

A preferred binder is low-substituted hydroxypropyl cellulose. When present it is present in a total amount of about 1 to 10%, e.g., about 2 to 5%, e.g., about 1% to 3%, in weight based on the total weight of the tablet.

In one embodiment, the binder is low-substituted hydroxypropyl cellulose which is present in a total amount of about 1 to 10%, e.g., about 2 to 5%, e.g., about 1% to 3%, in weight based on the total weight of the tablet.

Suitable lubricants for the dispersible tablets of the present invention include but are not limited to sodium stearyl fumarate, magnesium stearate, stearic acid, hydrogenated castor oil, calcium stearate, aluminum stearate, PEG 4000-8000, talc, glyceryl monostearate, glyceryl dibehenate (e.g., commercially available by Gattefossé under trademark Compritol^{®} 888 ATO), glyceryl palmito-stearic ester (e.g., commercially available by Gattefossé under trademark Precerol^{®}), hydrogenated cotton seed oil, castor seed oil, and any combination thereof. Preferably, sodium stearyl fumarate is used.

In the dispersible tablets of the present invention, the lubricant is present in a total amount from about 1% to about 15%, preferably about 1% to 7%, e.g., about 3% to 6%, e.g., about 3% to 5%, in weight based on the total weight of the tablet.

In one embodiment, the lubricant is sodium stearyl fumarate which is present in a total amount of about 1% to 7%, e.g., about 3% to 6%, e.g., about 3% to 5%, in weight based on the total weight of the tablet.

The dispersible tablet of the present invention optionally may include one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose. Examples of pharmaceutically acceptable excipients that additionally may be used in the present invention include but are not limited to one or more diluents, with the proviso that the diluent is not calcium phosphate tribasic, calcium phosphate dibasic anhydrous, or calcium phosphate dibasic dihydrate; glidants; surfactants; taste masking agent, with the proviso that the taste masking agent is not sucralose, or any combination thereof.

Preferably, the dispersible tablet of the present invention further comprises an additional pharmaceutically acceptable excipient that is a diluent, with the proviso that that the diluent is not calcium phosphate tribasic, calcium phosphate dibasic anhydrous, or calcium phosphate dibasic dihydrate. Suitable diluents for the dispersible tablets of the present invention include but are not limited to mannitol (including the alpha, beta and delta polymorphs), sorbitol, malodextrin, lactose (e.g., lactose monohydrate), microcrystalline cellulose, maltitol, xylitol, starch (e.g., corn, maize, or rice) or any combination thereof .

A preferred diluent is mannitol or microcrystalline cellulose, or a mixture of mannitol and microcrystalline cellulose. More preferably, the diluent is the delta polymorph of mannitol and/or microcrystalline cellulose.

The total amount of diluent may be in the range of about 20% to 70%, in particular, e.g., about 30% to 60%, e.g., about 35% to 55%, e.g., about 45% to 55%, e.g., about 45% to 50%, in weight based on the total weight of the tablet.

In one embodiment, the dispersible tablet of the present invention comprises an additional pharmaceutically acceptable excipient that is a diluent, with the proviso that that the diluent is not calcium phosphate tribasic, calcium phosphate dibasic anhydrous, or calcium phosphate dibasic dihydrate.

In one embodiment, the diluent is mannitol or microcrystalline cellulose. In one preferred embodiment, the diluent is mannitol and microcrystalline cellulose. In one preferred embodiment, the diluent is the delta polymorph of mannitol and/or microcrystalline cellulose.

In one embodiment, the tablet comprises an additional pharmaceutically acceptable excipient that is a diluent, wherein the diluent is present in a total amount in the range of about 30% to 60%, e.g., about 35% to 55%, e.g., about 45% to 55%, e.g., about 50% to 55%, in weight based on the total weight of the tablet, and wherein said diluent is mannitol and microcrystalline cellulose.

In one embodiment, the tablet comprises an additional pharmaceutically acceptable excipient that is a diluent, wherein the diluent is present in a total amount in the range of about 30% to 60%, e.g., about 35% to 55%, e.g., about 45% to 55%, e.g., about 50% to 55%, in weight based on the total weight of the tablet, and wherein said diluent is mannitol present in a total amount in the range of about 15% to 20% in weight based on the total weight of the tablet and microcrystalline cellulose present in a total amount in the range of about 25% to 35% in weight based on the total weight of the tablet.

The dispersible tablet of the present invention may comprise an additional pharmaceutically acceptable excipient that is a glidant. Suitable glidants for the dispersible tablets of the present invention include but are not limited to silica, colloidal silica (e.g., colloidal silica anhydrous), magnesium trisilicate, powdered cellulose, starch, talc and any combination thereof.

The total amount of glidant may be in the range of about 0.1% to 6%, in particular about 0.1% to 4%, e.g., about 0.1% to 2.5%, e.g., about 0.1 to 1.0%, in weight based on the total weight of the tablet.

In one embodiment, the dispersible tablet of the present invention comprises an additional pharmaceutically acceptable excipient that is a glidant.

In one embodiment, the tablet comprises an additional pharmaceutically acceptable excipient that is a glidant, wherein the total amount of glidant may be in the range of about 0.1% to 6%, in particular about 0.1% to 4%, e.g., about 0.1% to 2.5%, e.g., about 0.1 to 1.0%, in weight based on the total weight of the tablet.

The dispersible tablet of the present invention may comprise an additional pharmaceutically acceptable excipient that is a surfactant. Suitable surfactants for the dispersible tablets of the present invention include but are not limited to sodium lauryl sulfate, quaternary ammonium salts, polysorbates, sorbitan erters, poloxamer, vitamin E polyethylene glycol succinate, sucrose palmitate, docusate sodium, lecithin and any combination thereof.

The total amount of surfactant may be in the range of about 0.01% to 4%, in particular about 0.05% to 2.0%, e.g., about 0.05% to 1.0%, in weight based on the total weight of the tablet.

In one embodiment, the dispersible tablet of the present invention comprises an additional pharmaceutically acceptable excipient that is a surfactant.

In one embodiment, the dispersible tablet of the present invention comprises an additional pharmaceutically acceptable excipient that is a surfactant, wherein the total amount of surfactant may be in the range of about 0.01% to 4%, in particular about 0.05% to 2.0%, e.g., about 0.05% to 1.0%, in weight based on the total weight of the tablet.

The dispersible tablet of the present invention may comprise an additional pharmaceutically acceptable excipient that is a taste-masking agent, with the proviso that the additional pharmaceutical agent is not sucralose. A taste-masking agent can be a sweetener, a flavoring agent or a combination thereof.

A sweetener can be a sugar or a sugar substitute selected from lactose, mannitol, sucrose, glucose, thaumatin, neotame, tagatose, acesulfame potassium, saccharin sodium, aspartame, trehalose, saccharine or a combination thereof.

A flavoring agent is a substance capable of enhancing taste or aroma of a composition. Suitable flavoring agents can be selected from standard reference books, for example Fenaroli's Handbook of Flavor Ingredients, 3rd edition (1995). Suitable flavoring agents for the dispersible tablet of the present invention include but are not limited to (i.) natural flavors, which can be simulated with natural or synthetic agents or combinations thereof, such as almond, anise, apple, apricot, bergamot, blackberry, blackcurrant, blueberry, cacao, caramel, cherry, cinnamon, cranberry, eucalyptus, fig, ginger, grape, lemon, licorice, lime, malt, molasses, nutmeg, peach, pear, peppermint, pineapple, raspberry, rose, spearmint, strawberry, tangerine, tea, vanilla, wintergreen, etc., and (ii) synthetic flavors, such as tutti-frutti or bubblegum flavor. Flavoring agents can be used singly or in combinations of two or more.

One or more pharmaceutically acceptable excipients may be selected and used in the accordance with the present invention by one skilled in the art having regard to the particular desired properties of the dispersible tablet based on the present description and the experience and knowledge of one skilled in the art. In addition, the absolute amounts of each pharmaceutically acceptable excipient and the amounts relative to other excipients in the dispersible tablet of the present invention may be selected by one skilled in the art having regard to the particular desired properties of the dispersible tablet based on the present description and the experience and knowledge of one skilled in the art.

It will be appreciated that any given excipient may serve more than one function, e.g., as disintegrant, lubricant, binder, diluent, glidant and/or surfactant. For example, in a preferred embodiment, low-substituted hydroxypropyl cellulose is functioning as both a disintegrant and a binder.

The dispersible tablet of the present invention can be uncoated or film-coated. Suitable film coatings are known and commercially available or can be made according to known methods. Typically, film coating materials are hydrophilic polymers such as polyethylene glycol, polyvinylpyrrolidone, polyvinyl alcohol, hydroxypropylcellulose, hydroxymethylcellulose, and hydroxypropylmethylcellulose or the like. The film coating composition ingredients may include plasticizers in conventional amounts, as well as opacifiers and colorants. Typically, a film coating material is applied in such amount as to provide a film coating that ranges from about 1% to about 6% by weight of the total tablet. Dry mixtures such as Sepifilm or Opadry mixtures prepared by Colorcon Corp. may be used. These products are individually prepared dry pre-mixtures of film forming polymers, opacifiers, colorants and plasticizers which are further processed to aqueous film coating suspensions.

The film coating may be applied by conventional techniques in a suitable coating pan or fluidized bed apparatus using water and/or conventional organic solvents (e.g, methyl alcohol, ethyl alcohol, isopropyl alcohol), ketones (acetone), etc.

Preferably, the dispersible tablet of the present invention is uncoated.

In a preferred embodiment, the present invention relates to a dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) sodium starch glycolate in an amount of about 2% to 12% in weight based on the total weight of the tablet, (c) low-substituted hydroxypropyl cellulose in an amount of about 1% to 10 % in weight based on the total weight of the tablet, and (d) sodium stearyl fumarate in an amount of about 1% to 7 % in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

In a preferred embodiment, the present invention relates to a dispersible tablet comprising (a) (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 35% to 45% in weight based on the total weight of the tablet, (b) sodium starch glycolate in an amount of about 5% to 10% in weight based on the total weight of the tablet, (c) low-substituted hydroxypropyl cellulose in an amount of about 1% to 3% in weight based on the total weight of the tablet, and (d) sodium stearyl fumarate in an amount of about 3% to 5% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dihydrate or sucralose.

In one embodiment, the present invention relates to a dispersible tablet comprising granulates having an inner phase and outer phase, wherein the inner phase comprises *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant, at least one binder, and optionally any additional excipients, wherein the outer phase comprises at least one disintegrant, at least one lubricant, and optionally any additional excipients, and wherein the dispersible tablet comprises (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) at least one disintegrant in a total amount of about 1% to 25% in weight based on the total weight of the tablet, (c) at least one binder in a total amount of about 1% to 20% in weight based on the total weight of the tablet, (d) at least one lubricant in a total amount of about 1% to 15% in weight based on the total weight of the tablet, and optionally (e) one or more additional pharmaceutically acceptable excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

In a preferred embodiment, the present invention relates to a dispersible tablet comprising granulates having an inner phase and outer phase, wherein the inner phase comprises *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant sodium starch glycolate, at least one binder low-substituted hydroxypropyl cellulose, and optionally any additional excipients, wherein the outer phase comprises at least one disintegrant sodium starch glycolate, at least one lubricant sodium stearyl fumarate, and optionally any additional excipients, and wherein the dispersible tablet comprises (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) sodium starch glycolate in an amount of about 2% to 12% in weight based on the total weight of the tablet, (c) low-substituted hydroxypropyl cellulose in an amount of about 1% to 10 % in weight based on the total weight of the tablet, and (d) sodium stearyl fumarate in an amount of about 1% to 7 % in weight based on the total weight of the tablet, and optionally (e) one or more additional pharmaceutically acceptable excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

In a preferred embodiment, the present invention relates to a dispersible tablet comprising granulates having an inner phase and outer phase, wherein the inner phase comprises *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof, at least one disintegrant sodium starch glycolate, at least one binder low-substituted hydroxypropyl cellulose, and optionally any additional excipients, wherein the outer phase comprises at least one disintegrant sodium starch glycolate, at least one lubricant sodium stearyl fumarate, and optionally any additional excipients, and wherein the dispersible tablet comprises (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 35% to 45% in weight based on the total weight of the tablet, (b) sodium starch glycolate in an amount of about 5% to 10% in weight based on the total weight of the tablet, (c) low-substituted hydroxypropyl cellulose in an amount of about 1% to 3% in weight based on the total weight of the tablet, and (d) sodium stearyl fumarate in an amount of about 3% to 5% in weight based on the total weight of the tablet, and optionally (e) one or more additional pharmaceutically acceptable excipients, with the proviso that said additional pharmaceutically acceptable excipient in the inner phase or outer phase is not calcium phosphate tribasic, calcium phosphate dibasic anhydrous, calcium phosphate dibasic dihydrate or sucralose.

In accordance with the present invention, the dispersible tablets of the present invention show rapid disintegration in an aqueous medium such as water, soda or juice (e.g., fruit or vegetable juice). The rapid disintegration may be observed in standard tests known to one skilled in the art. The disintegration time is preferably measured according to the standard test for dispersible tablets described in European Pharmacopoeia Eighth edition, Supplement 8.2, page 811 (January 2014) in combination with European Pharmacopoeia, Eighth edition, Supplement 8.2, page 285-287, Section 2.9.1 (January 2014), which are both hereby incorporated by reference in their entirety. By "disintegration time" is meant the time that needs the dispersible tablet to disintegrate in water at room temperature in a disintegration time device. This test from the European Pharmacopoeia (January 2014) examines the disintegration time of tablets in water at 15 to 25 °C.

The rate and completion of disintegration may be observed visually. Disintegration is considered to be achieved when no residue remains on the screen of the test apparatus, or if there is residue remaining on the screen of the test apparatus, it consists of a soft mass having no palpably firm core, or only fragments of the insoluble coating remain on the screen.

The tablets of the present invention preferably have a disintegration time of 5 minutes or less, preferably 3 minutes or less, when measured according to the above test in water at a temperature of 15 to 25 °C. More preferably, the disintegration time is 90 seconds or less.

Preferably, the dispersible tablets of the present invention produce a smooth dispersion which passes through a sieve screen with a nominal mesh aperture of 710 µm after said tablets are completely dispersed in aqueous medium such as water, soda or juice (e.g., fruit or vegetable juice). This fineness of dispersion may be measured according to the standard test for dispersible tablets described in *European Pharmacopoeia* Eighth edition, Supplement 8.2, page 811 (January 2014), which is hereby incorporated by reference in its entirety.

In accordance with the present invention, the dispersible tablets of the present invention are dispersed in ingestible liquid, including but not limited to water, soda, and juice (e.g., fruit juice or vegetable juice), prior to administration to the subject. Preferably, the ingestible liquid is water. The resulting dispersed tablet may be administered to a subject or patient by oral ingestion or by administration via a feeding tube, preferably a gastronomy tube (G-tube) or PEG (percutaneous endoscopic gastrostomy) tube.

In one aspect, the dispersible tablets of the present invention is administered to a patient, such as a child or an adult suffering from a proliferative disease (e.g., a cancer), by (i) contacting said tablet with an ingestible liquid, (ii) allowing the tablet to disperse in the ingestible liquid to form a dispersed mixture, e.g., in 5 minutes or less, preferably 3 minutes or less, and (iii) ingesting the dispersed mixture.

In one embodiment, the present invention relates to a method of administering the dispersible tablet of the present invention to a patient in need thereof which comprises (i) contacting tablet with an ingestible liquid, (ii) dispersing the tablet in the ingestible liquid to form a dispersed mixture, e.g., in 5 minutes or less, preferably 3 minutes or less, and (iii) ingesting the dispersed mixture.

In one embodiment, the present invention relates to a method of administering the dispersible tablet of the present invention to a patient in need thereof which comprises instructing the subject in need thereof to (i) contact tablet with an ingestible liquid, (ii) disperse the tablet in the ingestible liquid to form a dispersed mixture, e.g., in 5 minutes or less, preferably 3 minutes or less, and (iii) ingest the dispersed mixture.

In another aspect, the dispersible tablets of the present invention may be administered to a patient in need thereof by (i) contacting said tablet with an ingestible liquid (ii) allowing the tablet to disperse in the ingestible liquid to form a dispersed mixture, e.g., in 5 minutes or less, preferably 3 minutes or less, and (iii) administering said dispersed mixture said patient using or via a feeding tube, preferably a gastronomy tube (G-tube) or PEG (percutaneous endoscopic gastrostomy) tube.

In one embodiment, the present invention relates to a method of administering the dispersible tablet of the present invention to a patient in need which comprises (i) contacting said tablet with an ingestible liquid (ii) dispersing the tablet in the ingestible liquid to form a dispersed mixture, e.g., in 5 minutes or less, preferably 3 minutes or less, and (iii) administering said dispersed mixture said patient using or via a feeding tube, preferably a gastronomy tube (G-tube) or PEG tube.

The dispersible tablets of the present invention may be dispersed in, e.g., 20 to 50 ml water with stirring.

In accordance with the present invention, it has been surprisingly found that these dispersible tablets having high drug loading (e.g., from about 5% to 50%, preferably about 35% to 45%, weight based on the total weight of the tablet) of Compound I and having dispersibility in aqueous medium, such as water, soda or juice (e.g., fruit or vegetable juice), in 5 minutes or less, preferably 3 minutes or less, may be obtained by wet granulation followed by compression methods.

Typically, wet granulation is not preferred for preparing dispersible tablets. Wet-granulation increases the cohesion of the active ingredient particles and increases the disintegration time of the final tablet which is not in accordance with patient compliance or the *European Pharmacopoeia* which requests a disintegration time of 3 minutes or less for a dispersible tablet. It has been surprisingly found that the specific use of Compound I with a disintegrant in a total amount of about 1% to 25% in weight based on the total weight of the tablet, a binder in a total amount of about 1% to 20% in weight based on the total weight of the tablet, and a lubricant in a total amount of about 1% to 15% in weight based on the total weight of the tablet produces a stable composition with high drug loading (e.g., from about 5% to 50%, preferably about 35% to 45%, weight based on the total weight of the tablet) and which disperses in aqueous medium in 5 minutes or less, preferably 3 minutes or less. Further, it has been surprisingly found that Compound I must be prepared as a dispersible tablet that does not include calcium phosphate tribasic, calcium phosphate dibasic anhydrous, calcium phosphate dibasic dihydrate or sucralose to achieve the foregoing physical and chemical properties and suitable stability.

In one aspect, the present invention relates to a process for producing a dispersible tablet of the present invention, comprising
(a) forming a granulate having an inner phase and an outer phase by
   (i.) wet granulating an inner phase comprising *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, at least one disintegrant in a total amount of about 1% to 15% in weight based on the total weight of the tablet, at least one binder in a total amount of about 1% to 20% in weight based on the total weight of the tablet, and optionally any additional pharmaceutically acceptable excipients;
   (ii.) adding at least one disintegrant in a total amount of about 1% to 10% in weight based on the total weight of the tablet, and optionally any additional pharmaceutically acceptable excipients to the inner phase formed in step (a)(i.) and mixing; and
   (iii.) adding at least one lubricant in a total amount of about 1% to 15% in weight based on the total weight of the tablet to the mixture formed in step (a)(ii.) and mixing; and
(b) forming the dispersible tablet by compressing the mixture obtained in step (a)(iii.),
with the proviso that any additional pharmaceutically acceptable excipient according to step (a)(i.) or (a)(ii.) is not calcium phosphate tribasic, calcium phosphate dibasic anhydrous, calcium phosphate dibasic dihydrate or sucralose. It is understood that steps (a)(ii) and (a)(iii) form the outer phase of the granulate.

Optionally, a film-coat may be applied to the tablet. In one embodiment, the process further comprises the step of applying a film-coat to the tablet.

Procedures which may be used may be conventional or known in the art or based on such procedures, e.g., those described in L. Lachman et al. The Theory and Practice of Industrial Pharmacy, 3rd Ed., 1986; H. Sucker et al., Pharmazeutische Technologie, Thieme, 1991, Hagers, Handbuch der Pharmazeutischen Praxis, 4th Ed. (Springer Verlag, 1971) and Remington's pharmaceutical Sciences, 13th Ed. (Mack Publ., Co., 1970) or later editions.

In one embodiment, the process described above includes one or more additional pharmaceutically acceptable excipient in step (a)(i.) that is a diluent, with the proviso that the diluent is not calcium phosphate tribasic, calcium phosphate dibasic anhydrous, or calcium phosphate dibasic dihydrate. Preferably, said diluent is mannitol or microcrystalline cellulose. More preferably, the diluent is mannitol and microcrystalline cellulose.

In one embodiment, the process described above includes one or more additional pharmaceutically acceptable excipient in step (a)(ii.) that is a diluent, with the proviso that the diluent is not calcium phosphate tribasic, calcium phosphate dibasic anhydrous, or calcium phosphate dibasic dihydrate. Preferably, said diluent is mannitol or microcrystalline cellulose. More preferably, the diluent is mannitol and microcrystalline cellulose.

In one embodiment, the process described above includes one or more additional pharmaceutically acceptable excipients in step (a)(i.) and step (a)(ii.) that is a diluent, with the proviso that the diluent is not calcium phosphate tribasic, calcium phosphate dibasic anhydrous, or calcium phosphate dibasic dihydrate. Preferably, said diluent is mannitol or microcrystalline cellulose. Preferably, the diluent is mannitol and microcrystalline cellulose. More preferably, the diluent in step (a)(i.) is mannitol and microcrystalline cellulose and the diluent in step (a)(ii.) is microcrystalline cellulose.

In one embodiment, the diluent in step (a)(i.) is mannitol which is present in a total amount of about 15% to 20% in weight based on the total weight of the tablet and microcrystalline cellulose which is present in an amount of about 15% to 20% in weight based on the total weight of the tablet, and the diluent in step (a)(ii.) is microcrystalline cellulose which is present in an amount of about 10% to 15% in weight based on the total weight of the tablet.

In one embodiment, the present invention relates to a process for producing a dispersible tablet of the present invention, comprising
(a) forming a granulate having an inner phase and an outer phase by
   (i.) wet granulating an inner phase comprising *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, sodium starch glycolate in a total amount of about 1% to 7% in weight based on the total weight of the tablet, low substituted hydroxypropyl cellulose in a total amount of about 1% to 10% in weight based on the total weight of the tablet, and at least one diluent;
   (ii.) adding sodium starch glycolate in a total amount of about 1% to 5% in weight based on the total weight of the tablet and at least one diluent to the inner phase formed in step (a)(i.) and mixing; and
   (iii.) adding sodium stearyl fumarate in a total amount of about 1% to 7% in weight based on the total weight of the tablet to the mixture formed in step (a)(ii.) and mixing; and
(b) forming the dispersible tablet by compressing the mixture obtained in step (a)(iii.),
with the proviso that the diluent according to step (a)(i.) or (a)(ii.) is not calcium phosphate tribasic, calcium phosphate dibasic anhydrous, or calcium phosphate dibasic dihydrate.

Preferably, the diluent in step (a)(i.) is mannitol and microcrystalline cellulose and the diluent in step (a)(ii.) is microcrystalline cellulose.

In one embodiment, the diluent in step (a)(i.) is mannitol which is present in a total amount of about 15% to 20% in weight based on the total weight of the tablet and microcrystalline cellulose which is present in an amount of about 15% to 20% in weight based on the total weight of the tablet, and the diluent in step (a)(ii.) is microcrystalline cellulose which is present in an amount of about 10% to 15% in weight based on the total weight of the tablet.

In one embodiment, the process further comprises the step of applying a film-coat to the tablet.

In a further embodiment, the present invention relates to a process for producing a dispersible tablet of the present invention, comprising
(a) forming a granulate having an inner phase and an outer phase by
   (i.) mixing Compound I or a pharmaceutically acceptable salt thereof in an amount of about 5% to 50% in weight based on the total weight of the tablet, at least one disintegrant in a total amount of about 1% to 15% in weight based on the total weight of the tablet, and optionally any additional pharmaceutically acceptable excipients in a high-shear granulator,
   (ii.) adding a solution comprising at least one binder to the mixture of step (a)(i.), subjecting the mixture to wetting and kneading in a high-shear granulator, wherein the binder is added to the mixture of step (a)(i.) in a total amount of about 1% to 20% in weight based on the total weight of the tablet
   (iii.) wet sieving said mixture of step (a)(ii.) in a screening mill,
   (iv.) drying the resulting mixture of step (a)(iii) in a fluidized bed dryer,
   (v.) screening the resulting dried mixture of step (a)(iv.) in a screening mill with oscillating bar,
   (vi.) adding sieved excipients comprising at least one disintegrant in an amount of about 1% to 5% in weight based on the total weight of the tablet, at least one lubricant in an amount of about 1% to 7% in weight based on the total weight of the tablet, and optionally any additional pharmaceutically acceptable excipients to the resulting mixture of step (v.) and mix in a diffusion mixture (tumble), and
(b) tableting the mixture from step (vi.) by compression using a conventional tableting press, preferably a rotary tableting press,
with the proviso that said additional pharmaceutically acceptable excipient according to steps (a)(i.), (a)(vi.) is not calcium phosphate tribasic, calcium phosphate dibasic anhydrous, calcium phosphate dibasic dihydrate or sucralose.

In one embodiment, the step (a)(i.) comprises one or more additional pharmaceutically acceptable excipients that is a diluent consisting of mannitol which is present in a total amount of about 15% to 20% in weight based on the total weight of the tablet and microcrystalline cellulose which is present in an amount of about 15% to 20% in weight based on the total weight of the tablet, and step (a)(i.) comprises one or more additional pharmaceutically acceptable excipients that is the diluent microcrystalline cellulose which is present in an amount of about 10% to 15% in weight based on the total weight of the tablet.

In one aspect, the present invention relates to a dispersible tablet made or obtained by the process described above. Preferably, the dispersible tablet is uncoated.

The physical and chemical stability may be tested in a conventional manner, e.g., the dispersible tablets may be tested as such by measurement of dissolution, friability, disintegration time, assay for Compound I degradation products, appearance and/or microscopy, e.g., after storage at room temperature, i.e., 25°C, and/or storage at 40°C.

The dispersible tablets may vary in shape and be, for example, round, oval, oblong, cylindrical or any other suitable shape. In a preferred embodiment of the present invention, the dispersible tablets obtained by the process described above are of round shape. The edges of the dispersible tablets may be beveled or rounded. More preferably, the dispersible tablets are of round shape or oval shape with beveled edges. The dispersible tablets may be scored, embossed or engraved.

The dispersible tablets of the present invention are preferably of round shape or oval shape, embossed, and with beveled edges.

The dispersible tablets of the present invention may be colored and/or marked as to impart an individual appearance and to make them instantly recognizable. The use of dyes can serve to enhance the appearance as well as to identify the dispersible tablets. Dyes suitable for use in pharmaceutical compositions or dosage forms typically include carotinoids, iron oxides or chlorophyll. The dispersible tablets of the present invention may be marked using an imprint code.

The hardness, or resistance to crushing, of tablets according to the present invention may be determined by standard tests. A device such as a Kraemer^{®} 3S tablet testing device may be used. This test determines the resistance to crushing of tablets, measured by the force needed to disrupt them by crushing.

The hardness of the tablets of the present invention varies according to the weight and diameter of the tablets and the compression force. For a tablet comprising about 50 mg of Compound I as active ingredient, having a diameter of about 7 mm, the hardness is preferably from about 25 N to 75N, preferably about 35 N to 65 N, most preferably about 50N and may be achieved by applying a compression force of about 5 to 11 kN. For a tablet comprising about 200 mg of Compound I as active ingredient, having a diameter of about 6.3 mm, the hardness is preferably from about 120N to 180N, preferably from about 130N to 170N, most preferably about 150N and may be achieved by applying a compression force of about 12 to 20 kN. For other tablet weights and diameters, the preferred hardness varies.

Thus, the advantageous properties of the dispersible tablets of the present invention may be demonstrated by the hardness and disintegration times of said tablets. Accordingly, in a preferred embodiment, the present invention comprises a dispersible tablet as defined above comprising 50 mg of Compound I or a pharmaceutically acceptable salt thereof, when compressed using a force of 5 to 11 kN with a 7 mm diameter die and standard round punches , has a hardness of 25 to 75 N and a disintegration time of 3 minutes or less. Accordingly, in a preferred embodiment, the present invention comprises a dispersible tablet as defined above comprising 200 mg of Compound I or a pharmaceutically acceptable salt thereof, when compressed using a force of 12 to 20 kN with a 16.0 x 6.3 mm die and standard ovaloid punches, has a hardness of 120 to 180 N and a disintegration time of 3 minutes or less.

The above statements of the present invention define the dispersible tablet in terms of the properties of a particular tablet made from the described pharmaceutical composition. However, it is clear that the invention is in no way thereby limited to tablets having such a weight, diameter, or hardness, or only to a production process involving the use of such a compression force. As discussed definition is rather given in order to clarify that the advantageous intrinsic properties of the formed tablet are including a rapid disintegration time in combination with a good degree of hardness.

The dispersible tablets of the present invention are useful for the treatment or prevention of a proliferative disorder, preferably a cancer.

The dispersible tablets of the present invention are particularly useful for the treatment or prevention of cancers including, for example, sarcoma, cancers of the lung, bronchus, prostate, breast (including sporadic breast cancers and sufferers of Cowden disease), pancreas, gastrointestine, colon, rectum, colon carcinoma, colorectal adenoma, thyroid, liver, intrahepatic bile duct, hepatocellular, adrenal gland, stomach, gastric, glioma, glioblastoma, endometrial, melanoma, kidney, renal pelvis, urinary bladder, uterine corpus, uterine cervix, vagina, ovary, multiple myeloma, esophagus, a leukemia, acute myelogenous leukemia, chronic myelogenous leukemia, lymphocytic leukemia, myeloid leukemia, brain, oral cavity and pharynx, larynx, small intestine, non-Hodgkin lymphoma, melanoma, villous colon adenoma, a neoplasia, a neoplasia of epithelial character, lymphomas, a mammary carcinoma, basal cell carcinoma, squamous cell carcinoma, actinic keratosis, head and neck, polycythemia vera, essential thrombocythemia, myelofibrosis with myeloid metaplasia, and Waldenstroem disease. Preferably, the dispersible tablets of the present invention are used for treatment of a proliferative disease.

Proliferative diseases mediated by the alpha-subunit of PI3K may include those showing overexpression or amplification of PI3K alpha, somatic mutation of PIK3CA or germline mutations or somatic mutation of PTEN or mutations and translocation of p85α that serve to up-regulate the p85-p110 complex. In a preferred embodiment, the cancer is a tumor and/or cancerous growth mediated by the alpha isoform of PI3K.

In one embodiment, the proliferative disease is a cancer selected from a cancer of the lung, bronchus, prostate, breast (including sporadic breast cancers and sufferers of Cowden disease), colon, rectum, colon carcinoma, colorectal adenoma, pancreas, gastrointestine, hepatocellular, stomach, gastric, ovary, squamous cell carcinoma, and head and neck.

In a preferred embodiment, the proliferative disease is a head and neck cancer.

In a preferred embodiment, the proliferative disease is breast cancer.

In a preferred embodiment, the dispersible tablets of the present invention are used for the treatment of a proliferative disorder, preferably a cancer.

In a further embodiment, the present invention relates to use of the dispersible tablet of the present invention for the treatment or prevention of a proliferative disease, preferably a cancer. Each of the embodiments set forth above are incorporated herein by reference.

In a further embodiment, the present invention relates to a method of treatment or prevention of a proliferative disease comprising administering to a patient in need thereof one or more dispersible tablets of the present invention that together comprise a therapeutically effective amount of Compound I or a pharmaceutically acceptable salt thereof. Each of the embodiments set forth above are incorporated herein by reference. Preferably, a subject or patient in need of said dispersible tablets of the present inventions are those subjects or patients suffering from a proliferative disorder, preferably a cancer.

The activity and characteristics of the dispersible tablets of the present invention may be indicated in standard clinical trials and/or animal trials.

Depending on age, patient condition, mode of administration, and type of disease, Compound I is orally administered at an effective daily dosage of about 1 to 6.5 mg/kg in adults or children. In a 70 kg body weight adult patient, Compound I is orally administered at a daily dosage of about 70 mg to 455 mg. It will be understood that the specific dose level for any particular patient will depend on a variety of factors including the age, body weight, general health, drug combination with one or more active drugs, type and severity of the disease. With the teachings of the present invention, one skilled in the art would have the experience and skill to select the proper dose level treatment of a specific patient.

In one aspect, the present invention further relates to a medicament package comprising dispersible tablets according to the present invention and printed instructions directing one or more dispersible tablets of Compound I or a pharmaceutically acceptable salt thereof be administered orally.

The following Example illustrates the invention described above, but is not, however, intended to limit the scope of the invention in any way. Other test models known as such to the person skilled in the pertinent art can also determine the beneficial effects of the claimed invention.

### Example 1: Dispersible tablet formulation (50 mg dispersible tablets with a disintegration time below 3 minutes)

| | Component | % per tablet | Amount per tablet (mg) |
|---|---|---|---|
| Internal Phase | Compound I | 39.4 | 50.00 |
| | Microcrystalline cellulose (Avicel PH101) | 18.1 | 23.00 |
| | Mannitol (Mannitol delta) | 18.1 | 23.00 |
| | Sodium starch glycolate (Type A) | 2.4 | 3.00 |
| | Low-substituted hydroxypropyl cellulose | 2.4 | 3.00 |
| | | | |
| External Phase | Microcrystalline cellulose (MCC PH102) | 12.5 | 15.86 |
| | Sodium stearyl fumarate | 4.0 | 5.08 |
| | Sodium starch glycolate (Type A) | 3.2 | 4.06 |
| | Total Core weight (mg) | | 127.00 |

The tablet is prepared by forming a granulate having an inner phase and an outer phase by (i.) wet granulating an inner phase comprising 50.00 mg of Compound I, 23.00 mg of microcrystalline cellulose (Avicel PH101), 23.00 mg of mannitol, 3.0 mg of sodium starch glycolate, and 3.00 mg of low-substituted hydroxypropyl cellulose, and then (ii) forming the outer phase by blending the resulting mixture with sieved 4.06 mg of sodium starch glycolate and 15.86 mg of microcrystalline cellulose (Avicel PH102) and then by blending the resulting mixture with sieved 5.08 mg sodium stearyl fumarate.

The tablet is obtained by tableting the mixture obtained in step (ii) above. The composition obtained in step (ii) above is compressed with a Fette^{®} 102i rotary tablet press using a compression force of about 5 to 11 kN (preferably about 8 kN) with a 7 mm diameter die and standard round punches.

The resulting tablet has the following properties:

| Test | Release specification |
|---|---|
| Tablet appearance | 7 mm diameter, round, flat, beveled edge with engraving |
| Tablet appearance | White |
| Hardness (20 units) | Target: 50 N |
| | Range of Individual: 25 to 75 N |
| | Range of mean: 35 to 65 N |
| Friability (after 100 rotations) | Max 0.8% (0 unit broken) |
| Disintegration time | All tested units less than or equal to 3 minutes Method: without discs, purified water at room temperature (between 15 and 25°C) |
| Average mass | 121.9 - 132.1 mg |
| Fineness of dispersion | Smooth dispersion which passes through a sieve screen with a nominal mesh aperture of 710 µm |

### Example 2: Dispersible tablet formulation (200 mg dispersible tablets with a disintegration time below 3 minutes)

| | Component | % per tablet | Amount per tablet (mg) |
|---|---|---|---|
| Internal Phase | Compound I | 39.4 | 200.00 |
| | Microcrystalline cellulose (Avicel PH101) | 18.1 | 92.00 |
| | Mannitol (Mannitol delta) | 18.1 | 92.00 |
| | Sodium starch glycolate (Type A) | 2.4 | 12.00 |
| | Low-substituted hydroxypropyl cellulose | 2.4 | 12.00 |
| | | | |
| External Phase | Microcrystalline cellulose (MCC PH102) | 12.5 | 63.44 |
| | Sodium stearyl fumarate | 4.0 | 20.32 |
| | Sodium starch glycolate (Type A) | 3.2 | 16.24 |
| | Total Core weight (mg) | | 508.00 |

The tablet is prepared by forming a granulate having an inner phase and an outer phase by (i.) wet granulating an inner phase comprising 200.00 mg of Compound I, 92.00 mg of microcrystalline cellulose (Avicel PH101), 92.00 mg of mannitol, 12.00 mg of sodium starch glycolate, and 12.00 mg of low-substituted hydroxypropyl cellulose, and then (ii) forming the outer phase by blending the resulting mixture with sieved 16.24 mg of sodium starch glycolate and 63.44 mg of microcrystalline cellulose (Avicel PH102) and then by blending the resulting mixture with sieved 20.32 mg sodium steary fumarate.

The tablet is obtained by tableting the mixture obtained in step (ii) above. The composition obtained in step (ii) above is compressed with a Fette^{®} 102i rotary tablet press using a compression force of about 12 to 20 kN (preferably about 16 kN) with 16.0 x 6.3 mm die and standard ovaloid punches.

The resulting tablet has the following properties:

| Test | Release specification |
|---|---|
| Tablet appearance | 16.0 x 6.3 mm ovaloid flat, beveled edge with engraving |
| Tablet appearance | White |
| Hardness (20 units) | Target: 150 N |
| | Range of Individual: 120 to 180 N |
| | Range of mean: 130 to 170 N |
| Friability (after 100 rotations) | Max 0.8% (0 unit broken) |
| Disintegration time | All tested units less than or equal to 3 minutes |
| | Method: without discs, purified water at room temperature (between 15 and 25°C) |
| Average mass | 492.8 - 523.2 mg |
| Fineness of dispersion | Smooth dispersion which passes through a sieve screen with a nominal mesh aperture of 710 µm |

### Example 3: Examples of Disintegration Times

The following table provides examples of disintegration times in minutes of tablets formulated according to Example 1 or Example 2:

| Test No. | Tablets Tested | Batch size that tested dispersible tablets were sampled from | Disintegration time |
|---|---|---|---|
| 1 | 50 mg dispersible tablet according to Example 1 | Approximately 1-2 kg | 1 minute |
| 2 | 50 mg dispersible tablet according to Example 1 | Approximately 20 kg | 1 minute |
| 3 | 200 mg dispersible tablets according to Example 2 | Approximately 1-2 kg | 1 minute |
| 4 | 200 mg dispersible tablets according to Example 2 | Approximately 74.3 kg | 2 minutes |

In the above tests, the specified tablets formulated according to Example 1 or Example 2 are evaluated to determine whether the tablets disintegrate within less than five minutes when placed in a liquid medium. The disintegration time of the tablets are evaluated according to the procedure set forth in European Pharmacopoeia, Eighth edition, Supplement 8.2, page 285-287, Section 2.9.1 (January 2014), which is hereby incorporated by reference in its entirety. No disks were used in these experiments.

Complete disintegration is defined as that state in which any residue of the tablet, except fragments of insoluble coating, remaining on the screen of the test apparatus is a soft mass having no palpably firm core.

A basket-rack assembly is used for these tests. The basket-rack assembly consists of 6 open-ended transparent tubes (each approximately 77.5 ± 2.5 mm long and having a diameter of approximately 21.85 ± 1.15 mm and a wall approximately 1.9 ± 0.9 mm thick) held by 2 plates having 6 holds equidistant from the center of the plate and equally spaced from another. Attached to the under surface of the lower plate is a woven stainless steel wire cloth, which has a plain square weave with 2.0 ± 0.2 mm mesh apertures and wit a wire diameter of 0.615 ± 0.045 mm. The parts of the apparatus are assembled and rigidly held by means of 3 bolts passing through the 2 plates. The basket-rack assembly is suspended from the raising and lowering device using a point on its axis. It is understood that the design of the basket-rack assembly may be varied provided that the specifications for the glass tubes and the screen mesh size are maintained.

In the disintegration test, one tablet is placed in each of the 6 tubes of the basket-rack assembly. The apparatus is operated using purified water at room temperature (approximately 15 to 25°C), as the immersion fluid. The tablets are evaluated for level and rate of disintegration by lifting the basket from the liquid. The results provided above reflect the maximum disintegration time from one tablet out of the six tablets in the disintegration test.
The present invention provides the following Enumerated Embodiments.

### Enumerated Embodiments

Enumerated Embodiment 1: A dispersible tablet comprising (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) at least one disintegrant in a total amount of about 1% to 25% in weight based on the total weight of the tablet, (c) at least one binder in a total amount of about 1% to 20% in weight based on the total weight of the tablet, (d) at least one lubricant in a total amount of about 1% to 15% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dehydrate or sucralose.
Enumerated Embodiment 2: The dispersible tablet according to Enumerated Embodiment 1, wherein *(S)*-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) or a pharmaceutically acceptable salt thereof is present in an amount of about 35 to 45% in weight based on the total weight of the tablet.
Enumerated Embodiment 3: The dispersible tablet according to Enumerated Embodiment 1 or Enumerated Embodiment 2, wherein at least one disintegrant is sodium starch glycolate or low-substituted hydroxypropyl cellulose.
Enumerated Embodiment 4: The dispersible tablet according to Enumerated Embodiment 1 or Enumerated Embodiment 2, wherein the disintegrant consists of sodium starch glycolate and low-substituted hydroxypropyl cellulose.
Enumerated Embodiment 5: The dispersible tablet according to any one of Enumerated Embodiments 1 to 4, wherein the binder is low-substituted hydroxypropyl cellulose.
Enumerated Embodiment 6: The dispersible tablet according to any one of Enumerated Embodiments 1 to 5, wherein the lubricant is sodium stearyl fumarate.
Enumerated Embodiment 7: The dispersible tablet comprising (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) sodium starch glycolate in an amount of about 2% to 12% in weight based on the total weight of the tablet, (c) low-substituted hydroxypropyl cellulose in an amount of about 1% to 10 % in weight based on the total weight of the tablet, and (d) sodium stearyl fumarate in an amount of about 1% to 7 % in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dehydrate or sucralose.
Enumerated Embodiment 8: The dispersible tablet according to any one of Enumerated Embodiments 1 to 7, wherein tablet comprises one or more additional pharmaceutically acceptable excipients selected from a diluent, with the proviso that the diluent is not calcium phosphate tribasic, calcium phosphate dibasic anhydrous, or calcium phosphate dibasic dihydrate; a glidant; a surfactant; a taste-masking agent, with the proviso that the taste-masking agent is not sucralose; or any combination thereof.
Enumerated Embodiment 9: The dispersible tablet according to Enumerated Embodiment 8, wherein the tablet comprises at least one diluent in a total amount of about 20% to 70% by weight based on the total weight of the tablet.
Enumerated Embodiment 10: The dispersible tablet according to Enumerated Embodiment 8 or 9, wherein the tablet comprises a diluent selected from mannitol, sorbitol, maltodextrin, lactose, microcrystalline cellulose, maltitol, xylitol, starch, or any combination thereof.
Enumerated Embodiment 11: The dispersible tablet according to any one of Enumerated Embodiments 1 to 10, wherein the disintegration time of the tablet is of about 5 minutes or less.
Enumerated Embodiment 12: The dispersible tablet according to any one of Enumerated Embodiments 1 to 10, wherein the tablet has a disintegration time of about 3 minutes or less.
Enumerated Embodiment 13: The dispersible tablet according to any one of Enumerated Embodiments 1 to 12 containing *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) in an amount of about 50 mg to 400 mg.
Enumerated Embodiment 14: A dispersible tablet according to any one of Enumerated Embodiments 1 to 10, wherein said tablet comprises 50 mg of *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof and wherein said tablet, when compressed using a force of 5 to 11 kN with a 7.0 mm diameter die and standard round punches, has a hardness of 25 to 75 N and a disintegration time of 3 minutes or less.
Enumerated Embodiment 15: A dispersible tablet according to any one of Enumerated Embodiments 1 to 10, wherein said tablet comprises 200 mg of *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof and wherein said tablet, when compressed using a force of 12 to 20 kN with a 16.0 x 6.3 mm die and standard ovaloid punches, has a hardness of 120 to 180 N and a disintegration time of 3 minutes or less.
Enumerated Embodiment 16: A process for the preparation of the dispersible tablet according to Enumerated Embodiment 1, which process comprises:
   (a) forming a granulate having an inner phase and an outer phase by
      (i.) wet granulating an inner phase comprising *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, at least one disintegrant in a total amount of about 1% to 15% in weight based on the total weight of the tablet, at least one binder in a total amount of about 1% to 20% in weight based on the total weight of the tablet, and optionally any additional pharmaceutically acceptable excipients;
      (ii.) adding at least one disintegrant in a total amount of about 1% to 10% in weight based on the total weight of the tablet, and optionally any additional pharmaceutically acceptable excipients to the inner phase formed in step (a)(i.) and mixing; and
      (iii.) adding at least one lubricant in a total amount of about 1% to 15% in weight based on the total weight of the tablet to the mixture formed in step (a)(ii.) and mixing; and
   (b) forming the dispersible tablet by compressing the mixture obtained in step (a)(iii.),
   with the proviso that any additional pharmaceutically acceptable excipient according to step (a)(i.) or (a)(ii.) is not calcium phosphate tribasic, calcium phosphate dibasic anhydrous, calcium phosphate dibasic dihydrate or sucralose.
Enumerated Embodiment 17: The process according to Enumerated Embodiment 16, wherein the wet granulating step (a)(i.) comprises at least one additional pharmaceutically acceptable excipient that is a diluent, with the proviso that the diluent is not calcium phosphate tribasic, calcium phosphate dibasic anhydrous, or calcium phosphate dibasic dihydrate.
Enumerated Embodiment 18: The process according to Enumerated Embodiment 17, wherein the diluent is mannitol and microcrystalline cellulose.
Enumerated Embodiment 19: The process according to Enumerated Embodiments 16 to 18, wherein step (a)(ii.) comprises adding at least one additional pharmaceutically acceptable excipient that is a diluent, with the proviso that the diluent is not calcium phosphate tribasic, calcium phosphate dibasic anhydrous, or calcium phosphate dibasic dihydrate.
Enumerated Embodiment 20: The process according to Enumerated Embodiment 19, wherein the diluent is microcrystalline cellulose.
Enumerated Embodiment 21: A dispersible tablet obtainable by the process of any one of Enumerated Embodiments 16-20.
Enumerated Embodiment 22: A method of administering the dispersible tablet of any one of Enumerated Embodiments 1 to 15 and 21 to a patient in need of said tablet which comprises (i) contacting tablet with an ingestible liquid (ii) allowing the tablet to disperse in the ingestible liquid to form a dispersed mixture and (iii) ingesting the dispersed mixture.
Enumerated Embodiment 23: A method of administering the dispersible tablet of any one of Enumerated Embodiments 1 to 15 and 21 to a patient in need of said tablet which comprises (i) contacting tablet with an ingestible liquid (ii) allowing the tablet to disperse in the ingestible liquid to form a dispersed mixture and (iii) administering said dispersed mixture said patient using or via a feeding tube.
Enumerated Embodiment 24: The method according to Enumerated Embodiment 23, wherein the patient in need of said tablet is a patient suffering from a proliferative disease.
Enumerated Embodiment 25: A dispersible tablet according to any one of Enumerated Embodiments 1 to 15 and 21 for use in treating or preventing a proliferative disease.
Enumerated Embodiment 26: The dispersible tablet according to Enumerated Embodiment 25, wherein the proliferative disease is a cancer selected from sarcoma, cancers of the lung, bronchus, prostate, breast (including sporadic breast cancers and sufferers of Cowden disease), pancreas, gastrointestine, colon, rectum, colon carcinoma, colorectal adenoma, thyroid, liver, intrahepatic bile duct, hepatocellular, adrenal gland, stomach, gastric, glioma, glioblastoma, endometrial, melanoma, kidney, renal pelvis, urinary bladder, uterine corpus, uterine cervix, vagina, ovary, multiple myeloma, esophagus, a leukemia, acute myelogenous leukemia, chronic myelogenous leukemia, lymphocytic leukemia, myeloid leukemia, brain, oral cavity and pharynx, larynx, small intestine, non-Hodgkin lymphoma, melanoma, villous colon adenoma, a neoplasia, a neoplasia of epithelial character, lymphomas, a mammary carcinoma, basal cell carcinoma, squamous cell carcinoma, actinic keratosis, head and neck, polycythemia vera, essential thrombocythemia, myelofibrosis with myeloid metaplasia, and Waldenstroem disease.

## Claims

1. A dispersible tablet comprising (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) at least one disintegrant in a total amount of about 1% to 25% in weight based on the total weight of the tablet, (c) at least one binder in a total amount of about 1% to 20% in weight based on the total weight of the tablet, (d) at least one lubricant in a total amount of about 1% to 15% in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dehydrate or sucralose.

2. The dispersible tablet according to claim 1, wherein *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof is present in an amount of about 35 to 45% in weight based on the total weight of the tablet.

3. The dispersible tablet according to claim 1 or claim 2, wherein:
(a) at least one disintegrant is sodium starch glycolate or low-substituted hydroxypropyl cellulose, optionally wherein the disintegrant consists of sodium starch glycolate and low-substituted hydroxypropyl cellulose;
(b) the binder is low-substituted hydroxypropyl cellulose; and/or
(c) the lubricant is sodium stearyl fumarate.

4. The dispersible tablet according to claim 1 comprising (a) *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, (b) sodium starch glycolate in an amount of about 2% to 12% in weight based on the total weight of the tablet, (c) low-substituted hydroxypropyl cellulose in an amount of about 1% to 10 % in weight based on the total weight of the tablet, and (d) sodium stearyl fumarate in an amount of about 1% to 7 % in weight based on the total weight of the tablet, and optionally (e) one or more additional pharmaceutically acceptable excipients, with the proviso that said additional pharmaceutically acceptable excipient is not calcium phosphate tribasic, calcium phosphate dibasic anhydrous, calcium phosphate dibasic dehydrate or sucralose.

5. The dispersible tablet according to any one of claims 1 to 4, wherein the tablet comprises one or more additional pharmaceutically acceptable excipients selected from a diluent, with the proviso that the diluent is not calcium phosphate tribasic, calcium phosphate dibasic anhydrous, or calcium phosphate dibasic dihydrate; a glidant; a surfactant; a taste-masking agent, with the proviso that the taste-masking agent is not sucralose; or any combination thereof, optionally wherein:
(a) the tablet comprises at least one diluent in a total amount of about 20% to 70% by weight based on the total weight of the tablet; and/or
(b) the tablet comprises a diluent selected from mannitol, sorbitol, malodextrin, lactose, microcrystalline cellulose, maltitol, xylitol, starch, or any combination thereof.

6. The dispersible tablet according to any one of claims 1 to 5, wherein the disintegration time of the tablet is of about 5 minutes or less or about 3 minutes or less.

7. The dispersible tablet according to any one of claims 1 to 6 containing *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) in an amount of about 50 mg to 400 mg.

8. A dispersible tablet according to any one of claims 1 to 5, wherein:
(a) said tablet comprises 50 mg of *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof and wherein said tablet, when compressed using a force of 5 to 11 kN with a 7.0 mm diameter die and standard round punches, has a hardness of 25 to 75 N and a disintegration time of 3 minutes or less; or
(b) said tablet comprises 200 mg of *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof and wherein said tablet, when compressed using a force of 12 to 20 kN with a 16.0 x 6.3 mm die and standard ovaloid punches, has a hardness of 120 to 180 N and a disintegration time of 3 minutes or less.

9. A process for the preparation of the dispersible tablet according to claim 1, which process comprises:
(a) forming a granulate having an inner phase and an outer phase by
(i.) wet granulating an inner phase comprising *(S)*-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) or a pharmaceutically acceptable salt thereof in an amount of about 5% to 50% in weight based on the total weight of the tablet, at least one disintegrant in a total amount of about 1% to 15% in weight based on the total weight of the tablet, at least one binder in a total amount of about 1% to 20% in weight based on the total weight of the tablet, and optionally any additional pharmaceutically acceptable excipients;
(ii.) adding at least one disintegrant in a total amount of about 1% to 10% in weight based on the total weight of the tablet, and optionally any additional pharmaceutically acceptable excipients to the inner phase formed in step (a)(i.) and mixing; and
(iii.) adding at least one lubricant in a total amount of about 1% to 15% in weight based on the total weight of the tablet to the mixture formed in step (a)(ii.) and mixing; and
(b) forming the dispersible tablet by compressing the mixture obtained in step (a)(iii.),
with the proviso that any additional pharmaceutically acceptable excipient according to step (a)(i.) or (a)(ii.) is not calcium phosphate tribasic, calcium phosphate dibasic anhydrous, calcium phosphate dibasic dihydrate or sucralose.

10. The process according to claim 9, wherein:
(a) the wet granulating step (a)(i.) comprises at least one additional pharmaceutically acceptable excipient that is a diluent, with the proviso that the diluent is not calcium phosphate tribasic, calcium phosphate dibasic anhydrous, or calcium phosphate dibasic dihydrate, optionally wherein the diluent is mannitol and microcrystalline cellulose; and/or
(b) step (a)(ii.) comprises adding at least one additional pharmaceutically acceptable excipient that is a diluent, with the proviso that the diluent is not calcium phosphate tribasic, calcium phosphate dibasic anhydrous, or calcium phosphate dibasic dihydrate, optionally wherein the diluent is microcrystalline cellulose.

11. A dispersible tablet obtainable by the process of claim 9 or claim 10.

12. A method of administering the dispersible tablet of any one of claims 1 to 8 and 11 to a patient in need of said tablet which comprises (i) contacting tablet with an ingestible liquid (ii) allowing the tablet to disperse in the ingestible liquid to form a dispersed mixture and (iii) ingesting the dispersed mixture or administering said dispersed mixture to said patient using or via a feeding tube.

13. The method according to claim 12, wherein the patient in need of said tablet is a patient suffering from a proliferative disease.

14. A dispersible tablet according to any one of claims 1 to 8 and 11 for use in treating or preventing a proliferative disease.

15. The dispersible tablet according to claim 14, wherein the proliferative disease is a cancer selected from sarcoma, cancers of the lung, bronchus, prostate, breast (including sporadic breast cancers and sufferers of Cowden disease), pancreas, gastrointestine, colon, rectum, colon carcinoma, colorectal adenoma, thyroid, liver, intrahepatic bile duct, hepatocellular, adrenal gland, stomach, gastric, glioma, glioblastoma, endometrial, melanoma, kidney, renal pelvis, urinary bladder, uterine corpus, uterine cervix, vagina, ovary, multiple myeloma, esophagus, a leukemia, acute myelogenous leukemia, chronic myelogenous leukemia, lymphocytic leukemia, myeloid leukemia, brain, oral cavity and pharynx, larynx, small intestine, non-Hodgkin lymphoma, melanoma, villous colon adenoma, a neoplasia, a neoplasia of epithelial character, lymphomas, a mammary carcinoma, basal cell carcinoma, squamous cell carcinoma, actinic keratosis, head and neck, polycythemia vera, essential thrombocythemia, myelofibrosis with myeloid metaplasia, and Waldenstroem disease.
